# EUROPEAN PATENT APPLICATION

(11) **EP 4 772 608 A1**
(43) Date of publication of application: **08.07.2026**
(21) Application number: 24860203.9
(22) Date of filing: 29.07.2024
(51) Int. Cl.: C12N 1/20, A61K 35/74, A61P 3/04, A23L 33/135, C12R 1/01

(54) **BACTEROIDES EGGERTHII KGMB05336 STRAIN AND USE THEREOF**

(30) Priority: 30.08.2023 KR 20230114198; 25.07.2024 KR 20240098538
(71) Applicant: AJOU UNIVERSITY INDUSTRY-ACADEMIC COOPERATION FOUNDATION, Gyeonggi-do 16499 (KR)
(72) Inventor: KIM, Soon Sun, Suwon-si, Gyeonggi-do 16517 (KR); EUN, Jung Woo, Suwon-si, Gyeonggi-do 16223 (KR); CHEON, Jae Youn, Suwon-si, Gyeonggi-do 16504 (KR); CHO, Hyo Jung, Suwon-si, Gyeonggi-do 16493 (KR); YOON, Moon Gyeong, Suwon-si, Gyeonggi-do 16397 (KR); WEON, Ji Hyang, Suwon-si, Gyeonggi-do 16226 (KR); LEE, Jung-Sook, Yuseong-gu, Daejeon 34140 (KR)
(74) Representative: Mewburn Ellis LLP
(86) International application number: PCT/KR2024/011033
(87) International publication number: WO 2025/048282

(57) **Abstract**

The present invention relates to the *Bacteroides eggerthii* KGMB05336 strain and a use thereof. Through the analysis of the microbiome in patients with non-alcoholic fatty liver disease that is caused by obesity, the *Bacteroides eggerthii* strain was identified as a significant strain. Oral administration of the *Bacteroides eggerthii* strain to animal models of obesity and non-alcoholic fatty liver disease caused by obesity resulted in body weight reduction, liver weight reduction, decrease of fat accumulation in visceral and liver tissues, and reduction in serum levels of AST (U/L), ALT (U/L), cholesterol (mg/dL), and glucose (mg/dL), thus demonstrating that the *Bacteroides eggerthii* strain can serve as a novel means for the prevention, improvement, or treatment of obesity and diseases caused by obesity, such as fatty liver, hepatomegaly, non-alcoholic fatty liver disease, and diabetes.

## Description

### Technical Field

The present disclosure relates to the *Bacteroides eggerthii* KGMB05336 strain and a use thereof.

### Background Art

Obesity, once a rare disease, is now a global epidemic. Currently, the World Health Organization estimates that 1.4 billion adults worldwide are overweight. Among these, 200 million men and 300 million women meet the criteria for obesity. Obesity is associated with a cluster of physiological disorders, including insulin resistance, type 2 diabetes, hypertension, dyslipidemia, cardiovascular disease, and metabolic syndrome. In the United States, obesity-related medical costs are estimated to be approximately 147 billion U.S. dollars annually. There is an urgent need for safe and effective interventions to address medical issues and costs associated with obesity.

Additionally, according to the [2019 Cause of Death Statistics] published by of Statistics Korea, the number of deaths due to liver disease in one year was 6,496, ranking eighth in the list of causes of death. These reports demonstrate that liver disease is the fifth leading cause of death, following cancer, heart disease, pneumonia, and cerebrovascular disease. In Korea, liver disease has become a major cause of death among economically active middle-aged individuals in their 40s and 50s. In particular, among individuals in their 40s, the mortality rate due to liver disease is 10.7 per 100,000 population, ranking third after cancer and suicide. Viral and alcoholic diseases are known causes of liver disease; however, with the increasing prevalence of obesity resulting from Westernized dietary habits, non-alcoholic fatty liver disease has been rapidly increasing. Liver disease caused by obesity may progress to liver cirrhosis, and once cirrhosis develops, it is difficult for the liver to recover to its original state even with treatment. It has been reported that liver cancer develops annually in approximately 1 to 3% of patients with liver cirrhosis. Therefore, it is considered that the most effective approach for preventing liver cirrhosis and liver cancer is to identify the underlying causes and prevent disease progression at the asymptomatic stage of obesity or non-alcoholic fatty liver disease.

Meanwhile, the term "microbiome" refers to the ecosystem of all microorganisms that coexist symbiotically within the human body and is known to perform various functions, including nutrient metabolism, metabolite production, and maintenance of immune homeostasis. The microbiome has a substantial impact on health and disease and is therefore sometimes referred to as the "second genome." Recently, diagnostic methods for various diseases utilizing the microbiome have been developed. In addition, studies have reported that patients' responses to treatment may vary depending on their baseline microbiome composition. Approximately 95% of all microorganisms present in the human body reside in the digestive tract, including the large intestine. Changes in the intestinal microbiome have been reported to contribute to liver injury and liver fibrosis through mechanisms including increased intestinal permeability, elevated levels of lipopolysaccharide (LPS) derived from intestinal bacteria, activation of Kupffer cells via stimulation of toll-like receptors (TLRs) in hepatocytes, increased levels of inflammatory cytokines such as TNF- α , and alterations in bile acid composition.

The inventors of the present disclosure discovered significant microbiome profiles by performing and analyzing 16S rRNA sequencing on fecal samples obtained from normal subjects and patients with obesity-induced non-alcoholic fatty liver disease, and verified the effects of the identified microbiome on obesity and fatty liver disease using animal models, thereby providing probiotic microorganisms that exhibit therapeutic effects on obesity and obesity-induced diseases.

### [Prior-Art Documents]

### [Patent Documents]

Korean Patent Application Publication No. 10-2015-0118084 (published on October 21, 2015)

Korean Patent Application Publication No. 10-2020-0093165 (published on August 5, 2020)

### Disclosure of the Invention

### Technical Goals

An object of the present invention is to develop a probiotic strain that exhibits therapeutic and ameliorating effects on obesity and obesity-induced diseases, and to provide a novel means for preventing, ameliorating, or treating obesity and obesity-induced diseases, such as fatty liver, hepatomegaly, non-alcoholic fatty liver disease, and diabetes, by using the probiotic strain.

### Technical Solutions

The present disclosure provides a pharmaceutical composition for preventing or treating obesity or an obesity-induced disease, including the *Bacteroides eggerthii* KGMB05336 strain, or a culture or dried product thereof.

In addition, the present disclosure provides a probiotic composition for preventing or ameliorating obesity or an obesity-induced disease, including the *Bacteroides eggerthii* KGMB05336 strain, or a culture or dried product thereof.

In addition, the present disclosure provides a health functional food composition for preventing or ameliorating obesity or an obesity-induced disease, including the *Bacteroides eggerthii* KGMB05336 strain, or a culture or dried product thereof.

### Advantageous Effects

According to the present disclosure, as a result of analyzing the microbiome of patients with a non-alcoholic fatty liver disease, which is a disease caused by obesity, the *Bacteroides eggerthii* strain was identified as a significant strain, and as a result of oral administration of the *Bacteroides eggerthii* strain to animal models with obesity and a non-alcoholic fatty liver disease caused by obesity, it was found that body weight was reduced, liver weight was reduced, a degree of fat accumulation in the intestines and liver was reduced, and levels of serum AST (U/L), ALT (U/L), cholesterol (mg/dL), and glucose (mg/dL) were improved, suggesting that the *Bacteroides eggerthii* strain may be provided as a new means for preventing, ameliorating, or treating obesity and obesity-induced diseases, such as fatty liver, hepatomegaly, non-alcoholic fatty liver disease, and diabetes.

### Brief Description of Drawings

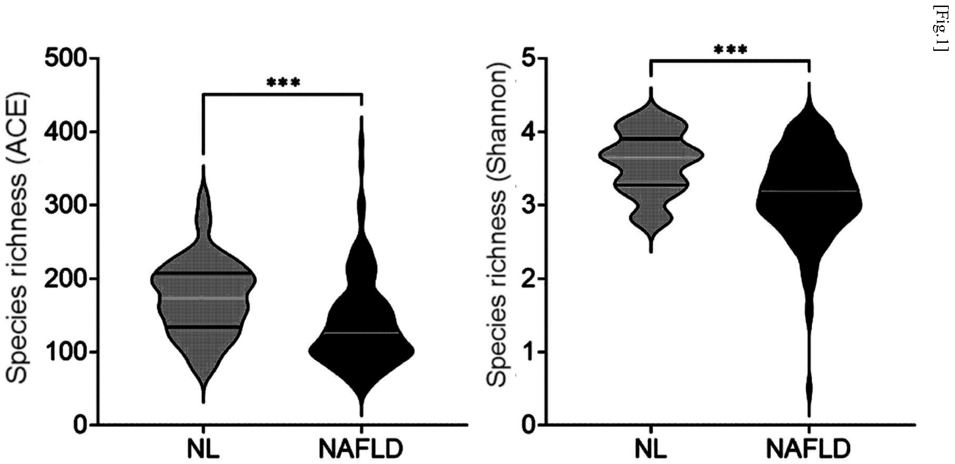

FIG. 1 shows results of analyzing species richness based on results from the microbiome of a normal control group (NL) and a non-alcoholic fatty liver disease (NAFLD) group.

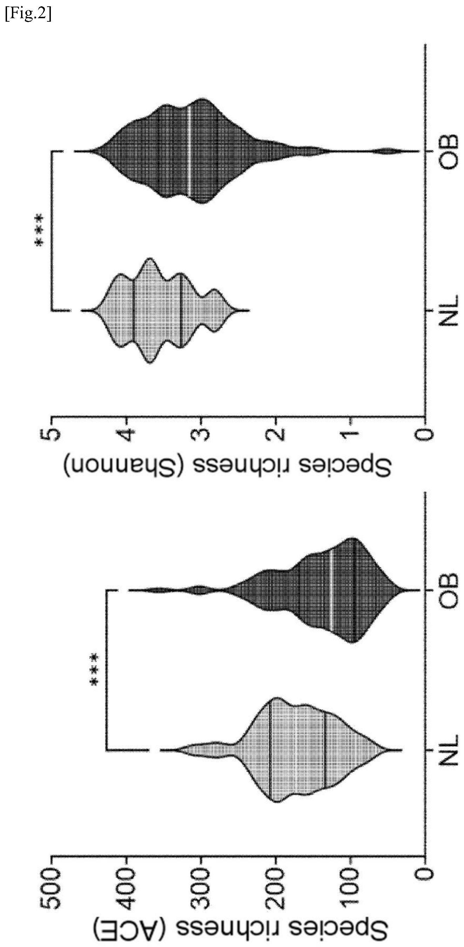

FIG. 2 shows results of analyzing species richness based on results from the microbiome of a normal control group (NL) and an obese group (OB).

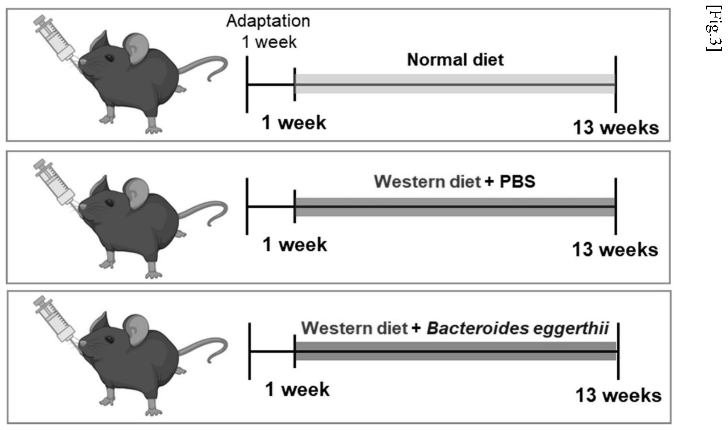

FIG. 3 is schematic diagrams of an experiment to establish an animal model with a non-alcoholic fatty liver disease caused by obesity and evaluate an effect of the *Bacteroides eggerthii* strain.

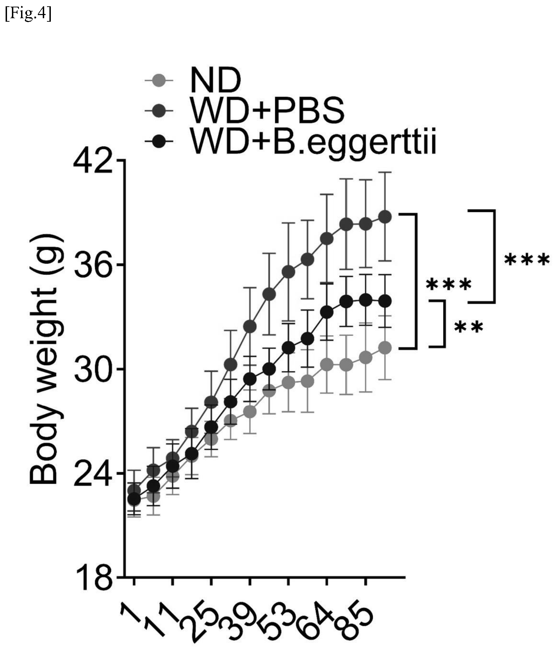

FIG. 4 shows a result of measuring a change in a body weight of a normal diet group (ND) that was fed a normal diet, a negative control group (WD+PBS) that was fed only a Western diet, and an experimental group (WD+B.eggerttii) that was fed a Western diet while receiving *Bacteroides eggerthii* , according to the present disclosure.

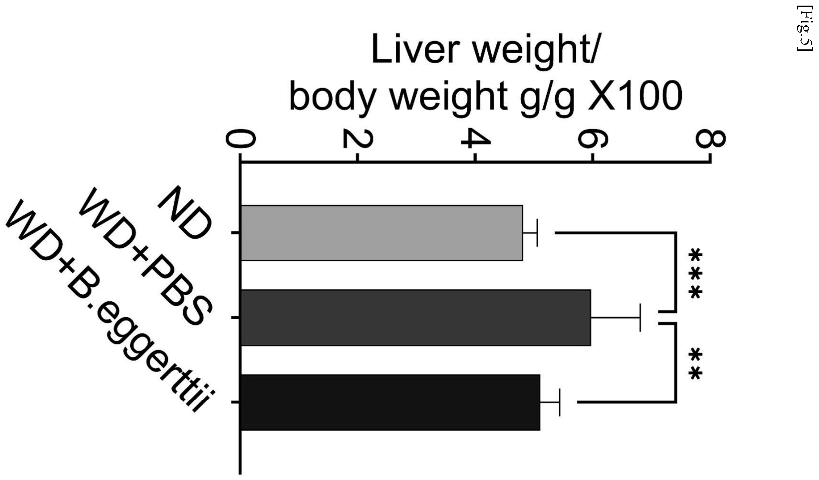

FIG. 5 shows a result of measuring a liver weight of a normal diet group (ND) that was fed a normal diet, a negative control group (WD+PBS) that was fed only a Western diet, and an experimental group (WD+B.eggerttii) that was fed a Western diet while receiving *Bacteroides eggerthii* , according to the present disclosure.

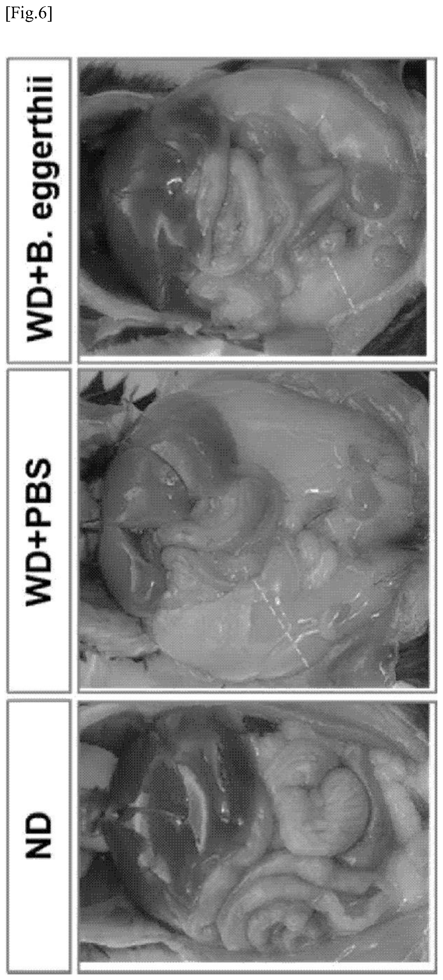

FIG. 6 shows results of measuring a degree of visceral fat accumulation of a normal diet group (ND) that was fed a normal diet, a negative control group (WD+PBS) that was fed only a Western diet, and an experimental group (WD+B.eggerttii) that was fed a Western diet while receiving *Bacteroides eggerthii* , according to the present disclosure.

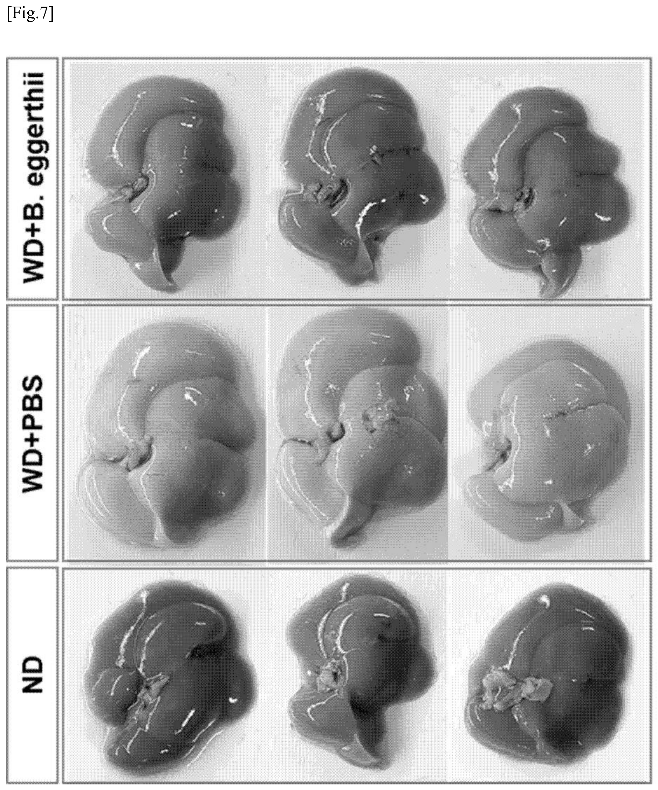

FIG. 7 shows results of measuring a degree of liver fat accumulation of a normal diet group (ND) that was fed a normal diet, a negative control group (WD+PBS) that was fed only a Western diet, and an experimental group (WD+B.eggerttii) that was fed a Western diet while receiving *Bacteroides eggerthii* , according to the present disclosure.

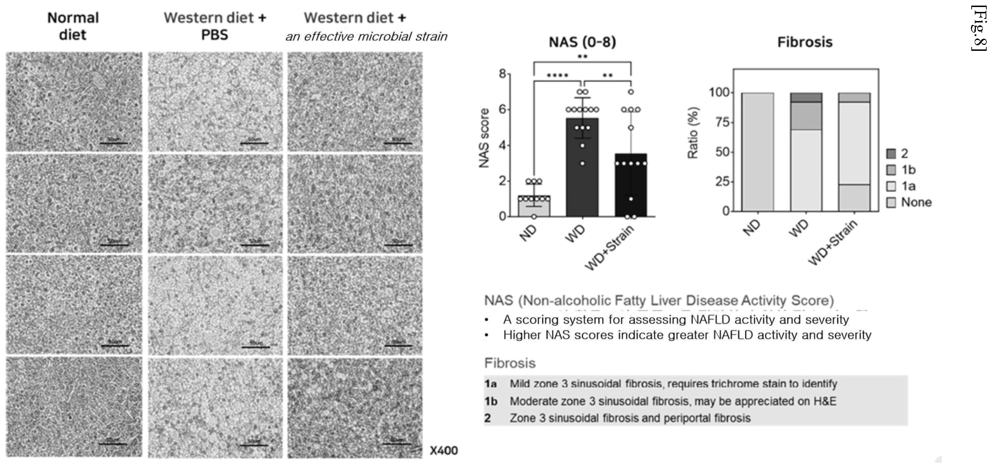

FIG. 8 shows results of evaluating pathological changes in liver tissues of a normal diet group (ND) that was fed a normal diet, a negative control group (WD+PBS) that was fed only a Western diet, and an experimental group (WD+B.eggerttii) that was fed a Western diet while receiving *Bacteroides eggerthii* , according to the present disclosure.

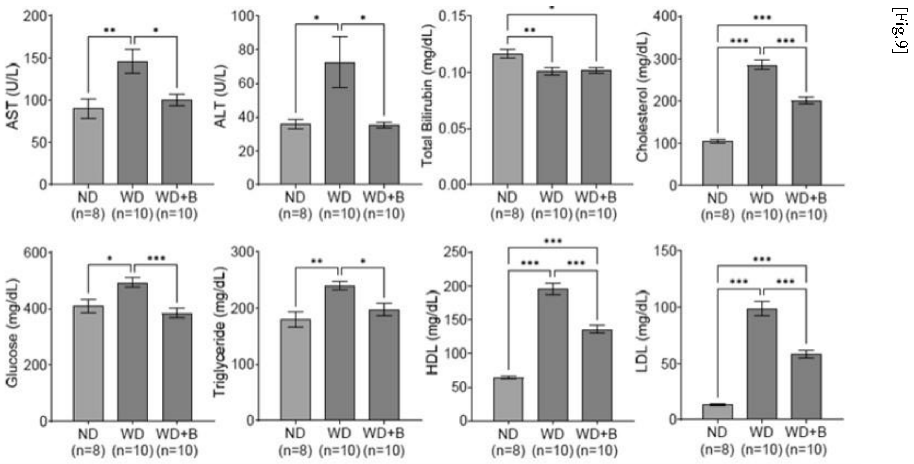

FIG. 9 shows results of a blood test of a normal diet group (ND) that was fed a normal diet, a negative control group (WD+PBS) that was fed only a Western diet, and an experimental group (WD+B.eggerttii) that was fed a Western diet while receiving *Bacteroides eggerthii ,* according to the present disclosure.

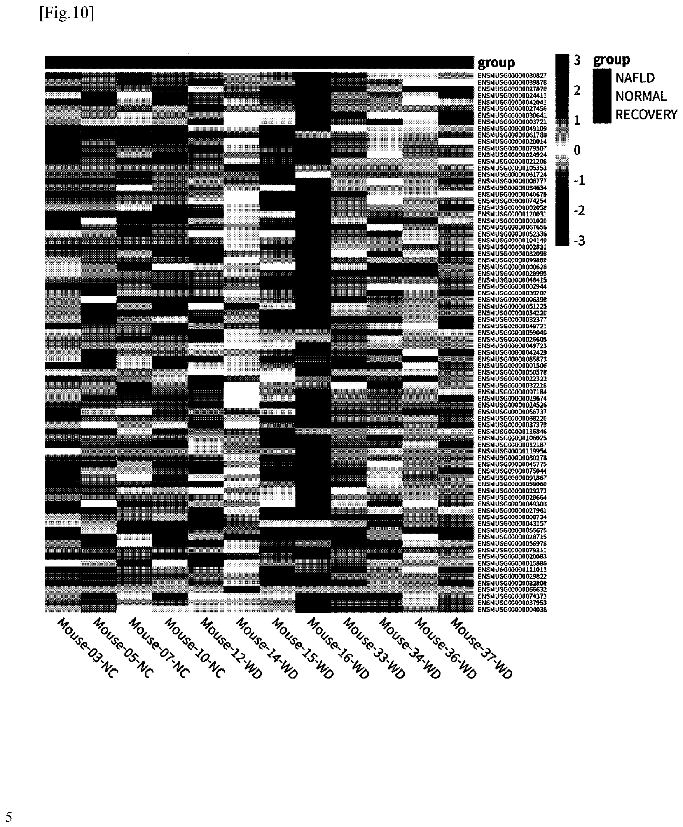

FIG. 10 shows a result of heatmap analysis conducted to analyze a correlation between genes whose expression decreased upon administration of *Bacteroides eggerthii* among genes whose expression increased in a Western diet group compared to a normal diet group and expression of fecal microbiome mRNA, according to the present disclosure.

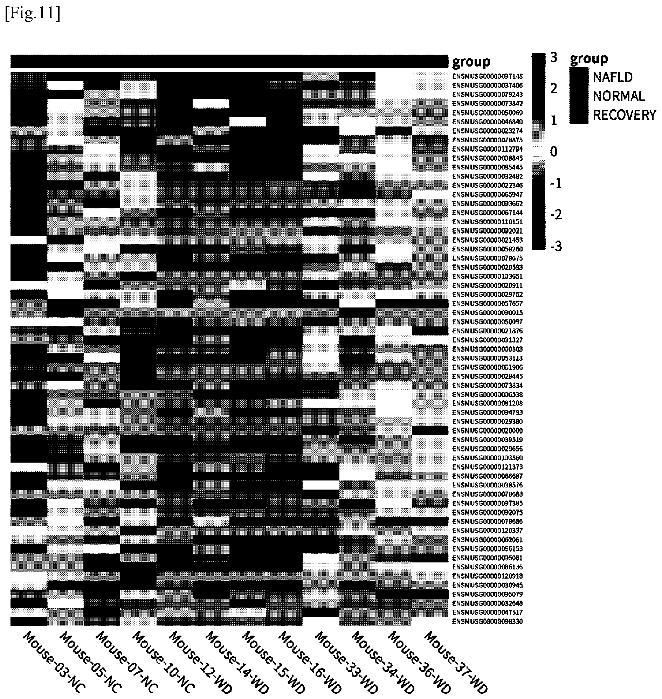

FIG. 11 shows a result of heatmap analysis conducted to analyze a correlation between genes whose expression increased upon administration of *Bacteroides eggerthii* among genes whose expression decreased in a Western diet group compared to a normal diet group and expression of fecal microbiome mRNA, according to the present disclosure.

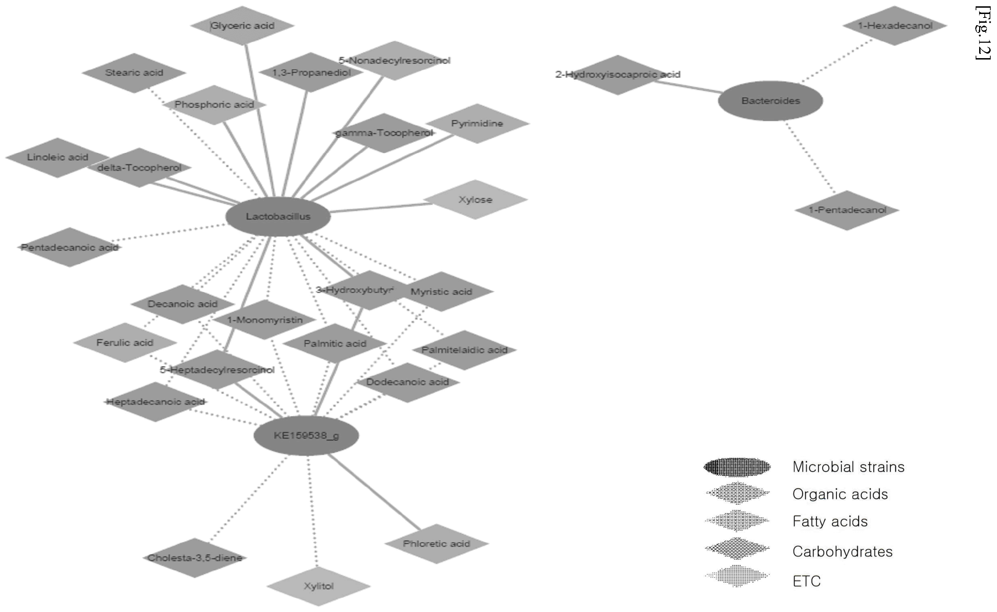

FIG. 12 shows a result of analyzing a network of metabolites presenting a significant correlation with the microbiome, which increased in proportion in a Western diet group compared to a normal diet group but decreased upon administration of *Bacteroides eggerthii* , according to the present disclosure.

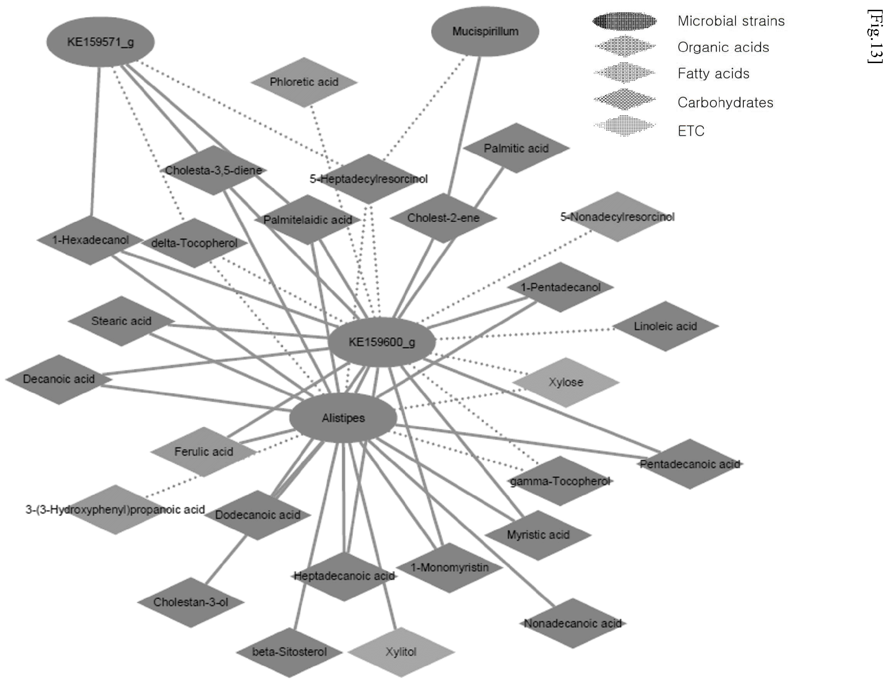

FIG. 13 shows a result of analyzing a network of metabolites presenting a significant correlation with the microbiome, which decreased in proportion in a Western diet group compared to a normal diet group, but increased upon administration of *Bacteroides eggerthii* , according to the present disclosure.

### Best Mode for Carrying Out the Invention

The terms used in the present specification have been selected from general terms that are currently widely used as much as possible while considering the functions in the present disclosure, but these may vary depending on the intention of those skilled in the art or precedents or the emergence of new technologies. Additionally, in certain cases, there are terms arbitrarily selected by the applicant, and in such cases, meanings thereof will be set forth in detail in the detailed description of the disclosure. Therefore, the terms used herein should be defined based on the meaning of the terms and the overall content of the present disclosure, rather than simply the names of the terms.

Unless otherwise defined, all terms used herein, including technical or scientific terms, have the same meaning as commonly understood by those skilled in the art to which the present disclosure pertains. Terms defined in commonly used dictionaries should be interpreted as having a meaning consistent with the meaning in the context of the relevant art, but not be interpreted in an idealized or overly formal sense, unless explicitly defined in this application.

### Hereinafter, the present disclosure will be described in more detail.

The present disclosure provides a pharmaceutical composition for preventing or treating obesity or an obesity-induced disease, including the *Bacteroides eggerthii* KGMB05336 strain, or a culture or dried product thereof.

The obesity-induced disease may be any one or more selected from fatty liver, hepatomegaly, non-alcoholic fatty liver disease, and diabetes.

When the pharmaceutical composition is administered orally, it has the effect of reducing body weight, improving fatty liver, and improving serum AST (U/L), ALT (U/L), cholesterol (mg/dL), and glucose (mg/dL) levels.

The pharmaceutical composition of the present disclosure may be prepared in the form of a unit dose by formulating with a pharmaceutically acceptable carrier or prepared by encapsulating in a multi-capacity container, in accordance with a method that may be easily carried out by those with ordinary skill in the art to which the disclosure pertains.

The pharmaceutically acceptable carriers are those that are commonly used in preparation, including, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may further include lubricants, humectants, sweeteners, flavoring agents, emulsifiers, suspensions, and preservatives, in addition to the above ingredients.

In the present disclosure, the content of the additives included in the pharmaceutical composition is not particularly limited and may be appropriately adjusted within a content range used in the conventional preparation.

The pharmaceutical composition may be formulated in the form of one or more external agents selected from the group consisting of injectable formulations such as aqueous solutions, suspensions, and emulsions, pills, capsules, granules, tablets, creams, gels, patches, sprays, ointments, plasters, lotions, liniments, pastas, and cataplasmas.

The pharmaceutical composition of the present disclosure may include a pharmaceutically acceptable carrier and diluent that are additionally present for formulation. The pharmaceutically acceptable carriers and diluents include, but are not limited to, excipients such as starch, sugars, and mannitol, fillers and extenders such as calcium phosphate, cellulose derivatives such as carboxymethylcellulose and hydroxypropylcellulose, binders such as gelatin, alginate, and polyvinyl pyrrolidone, lubricants such as talc, calcium stearate, hydrogenated castor oil, and polyethylene glycol, disintegrating agents such as povidone and crospovidone, and surfactants such as polysorbate, cetyl alcohol, and glycerol. The pharmaceutically acceptable carriers and diluents may be biologically and physiologically friendly to the subject. Examples of diluents may include, but are not limited to, brine, water-soluble buffers, solvents, and/or dispersion media.

The pharmaceutical composition of the present disclosure may be administered orally or parenterally (e.g., applied intravenously, subcutaneously, intraperitoneally, or topically) depending on the desired method. When administered orally, it may be formulated as tablets, troches, lozenges, water-soluble suspensions, oil-based suspensions, powder preparation, granules, emulsions, hard capsules, soft capsules, syrups, and elixirs. When administered parenterally, it may be formulated as injection solutions, suppositories, powders for respiratory inhalation, aerosols for sprays, ointments, powders for application, oils, and creams.

The dosage of the pharmaceutical composition of the present disclosure may vary depending on the patient's condition, weight, age, sex, health status, dietary constitution specificity, nature of the preparation, severity of disease, administration time for composition, method of administration, duration or interval of administration, excretion rate, and drug form, and may be appropriately selected by a person skilled in the art. For example, it may range from about 0.1 to 10,000 mg/kg but is not limited thereby, and it may be administered once or several times a day.

The pharmaceutical composition may be administered orally or parenterally (e.g., applied intravenously, subcutaneously, intraperitoneally, or topically) depending on the desired method. The pharmaceutically effective amount and effective dosage of the pharmaceutical composition of the present disclosure may vary by the preparation method of the pharmaceutical composition, type of administration, administration time, and administration route, and those with ordinary skill in the art may easily determine and prescribe the effective dosage for the desired treatment. The administration of the pharmaceutical composition of the present disclosure may be conducted once a day or in several divided doses.

In addition, the present disclosure provides a probiotic composition for preventing or ameliorating obesity or obesity-induced diseases, including the *Bacteroides eggerthii* KGMB05336 strain, or a culture or dried product thereof.

In the present disclosure, the probiotic composition may be prepared and administered in various formulations and methods according to methods known in the art. For example, it may be prepared and administered in the form of powders, liquids and solutions, tablets, capsules, syrups, suspensions, or granules by mixing with commonly used carriers. For example, the carrier may include, but is not limited to, binders, lubricants, disintegrants, excipients, solubilizers, dispersants, stabilizers, suspending agents, pigments, and fragrances. In addition, the dosage may be appropriately selected depending on the absorption rate, inactivation rate, and excretion rate of the active ingredient in the body as well as age, sex, species, condition, and severity of the disease of a recipient.

The method of culturing the strain of the present disclosure may be carried out according to a method commonly used in the art, but is not limited to a specific method.

When the strain or the culture of the strain or the concentrate of the culture solution of the strain obtained in the culturing of the strain of the present disclosure is used as an additive, the strain or the culture of the strain or the concentrate of the culture solution of the strain may be added as it is or used together with other additives, and may be used appropriately according to a conventional method. The amount of the active ingredient to be mixed may be appropriately determined depending on the purpose of use.

When the probiotics of the present disclosure are used as food additives, the probiotics may be added as is or used together with other foods or food ingredients, and may be used appropriately according to conventional methods. The amount of active ingredients mixed may be appropriately determined depending on its intended use (prevention, health or therapeutic treatment). In general, when manufacturing food or beverage, the probiotics of the present disclosure are added in an amount of 15 parts by weight or less, preferably 10 parts by weight or less, relative to the raw material. However, in the case of long-term consumption for the purpose of health and hygiene or health control, the mixed amount may be below the above range, and the mixed amount may also be used in an amount above the range since there is no problem in terms of safety.

There are no special restrictions on the types of the foods. Examples of foods to which the probiotics may be added include bread, candy, snacks, confectionery, gum, dairy products including ice cream, various soups, beverages, tea, drinks, and vitamin complexes, and include all health foods in the conventional sense.

In addition, the present disclosure provides a health functional food composition for preventing or ameliorating obesity or obesity-induced diseases, including the *Bacteroides eggerthii* KGMB05336 strain, or a culture or dried product thereof.

The present disclosure may be used generally as a food product that is conventionally used.

The food composition of the present disclosure may be used as a health functional food. The term "health functional food" as used herein refers to a food manufactured and processed using raw materials or ingredients that have useful functionality for the human body in accordance with the Health Functional Food Act, and the term "functionality" as used herein refers to consumption for the purpose of deriving useful effects for health purposes such as adjusting nutrients or physiological actions on the structure and function of the human body.

The food composition of the present disclosure may include conventional food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

The food composition of the present disclosure may be manufactured and processed in the form of tablets, capsules, powder, granules, liquids, and pills.

For example, hard capsules among health functional foods in the form of capsules may be prepared by mixing and filling the composition according to the present disclosure in conventional hard capsules along with additives such as excipients, and the soft capsules may be manufactured by mixing the composition according to the present disclosure with the additives such as excipients and then filling the same in capsule bases such as gelatin. The soft capsule may contain, if necessary, plasticizers such as glycerin or sorbitol, colorants, and preservatives.

The definition of terms for the excipient, binder, disintegrant, lubricant, flavor enhancer, and flavoring agent is described in documents known in the art and includes those having the same or similar functions. The type of food is not particularly limited and includes all health functional foods in the ordinary sense.

The term "prevention" as used herein refers to any action of suppressing or delaying diseases by administering the composition according to the present disclosure. The term "treatment" as used herein refers to any action that ameliorates or favorably changes the symptoms of diseases by administering the composition according to the present disclosure. The term "amelioration" as used herein refers to any action of making the bad condition of diseases better by having an individual administer or ingest the composition of the present disclosure.

As used herein, the treatment refers to an approach to obtain a beneficial or desirable clinical outcome, which for the purposes of the present disclosure may include, without limitation, alleviation of symptoms, reduction in the extent of the disease, stabilization (i.e., not worsening) of the disease state, delay or reduction in the rate of disease progression, amelioration or temporal palliation and alleviation (partial or total) of the disease state, and whether it is detectable or undetectable, and may also refer to an increase in survival compared to the expected survival rate if no treatment is received. Treatment refers to both therapeutic treatment and preventive or prophylactic measures. The treatments include not only the treatment required for disorders to be prevented but also the treatment required for disorders that have already occurred. The term "palliating" a disease as used herein refers to reducing the extent of the disease state and/or unfavorable clinical signs and/or slowing or extending the time course of progression, compared to a case without undergoing treatment.

### Modes for Carrying Out the Invention

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

### Example 1. Discovery of microbiome markers

### 1-1. Subjects

To analyze the microbiome associated with obesity and diseases caused by obesity, subjects for microbiome analysis were selected from among patients with non-alcoholic fatty liver disease, which is a disease caused by obesity among patients diagnosed with obesity.

Fecal and clinical information was obtained from patients with non-alcoholic fatty liver disease who visited Ajou University Hospital from October 12, 2018 to July 29, 2021, as well as from normal controls. Among adults aged 19 years or older, those who visited the outpatient clinic of the gastroenterology department or were enrolled through a recruitment notice agreed to the collection of blood and fecal samples (research consent form obtained), and the experiment was conducted under research approval (AJOUIRB-SUR-2019-096). Those who did not meet the following exclusion criteria were selected.
1. Those who are positive for HBV, HCV, HIV
2. Those with autoimmune hepatitis, Wilson's disease, and primary biliary cholangitis
3. Those who are being treated for liver cancer or other organ cancer or who were diagnosed with such diseases within the past 5 years
4. Those who have an active infection or are receiving antibiotics
5. Those with excessive drinking habits (men: 30g/day, women: 20g/day)

Fecal samples and clinical information from 50 healthy adult normal controls (NL) and 148 patients diagnosed with non-alcoholic fatty liver disease (NAFLD) were obtained from Ajou University Hospital. Among the 148 patients, patients with a BMI of ≥ 25 kg/m² were classified into an obese group (OB) and recognized as patients with a non-alcoholic fatty liver disease caused by obesity. The subjects were provided with a stool collection bag for stool collection and were instructed regarding collection and storage methods. Stool collected within 1 to 3 days of outpatient visit was frozen and stored at home, and stool samples (5 g) were collected from the subjects at the next outpatient visit. When collecting fecal samples, the following clinical information was additionally collected: presence of non-alcoholic fatty liver disease, liver fibrosis test, body fat analysis, liver biopsy, sex, age, height, body weight, concomitant diseases, dietary habits, drinking/smoking history, medication history, and blood test results (CBC, biochemical parameters, lipid panel, insulin level, c-peptide, HbA1C). The collected clinical information and blood and fecal samples were deposited in the Human Resources Bank and stored frozen.

### 1-2. Analysis of microbiome

To select microbiome markers associated with the non-alcoholic fatty liver disease and obesity from the collected fecal samples, 16S ribosomal RNA (rRNA) sequencing and metagenomics sequencing were used. The fecal samples were diluted in 10 mL of phosphate-buffered saline for 24 hours, filtered through a cell strainer, and centrifuged at 10,000Xg for 10 min at 4°C to separate the pellet containing bacteria and the supernatant containing EV. The EV sample was sterilized and purified through a 0.22 µm filter to completely remove bacteria and foreign substances. Bacteria and EV samples were boiled at 100°C for 40 min, and then the supernatant was collected after 30 min and centrifuged at 4°C, 18214 Xg to remove any remaining suspended particles and waste. Next, DNA was extracted from the bacterial and bacterial EV membranes using the DNeasy Power Soil kit (QIAGEN).

To analyze the microbiome from the DNA of microorganisms extracted from the samples, amplicon sequencing was performed on the 16S rRNA of each strain using Illumina's MiSeq next-generation sequencing platform. Bacterial genomic DNA was amplified with primers targeting the V3-V4 hypervariable region of 16S rDNA (16S_V3_F 5'-TCGTCGGCAGCGTCAGATGTGTATAAGAGACAGCCTACGGGNGGCWGCAG-3' SEQ ID NO: 1 and 16S_V4_R 5'-GTCTCTCGTGGGCTCGGATCCGT-3' SEQ ID NO: 2). Cycling conditions were at 95°C for 3 min (35 cycles at 95°C for 30 s, at 58°C for 30 s, and at 72°C for 30 s; at 72°C for 5 min). Libraries were prepared using polymerase chain reaction (PCR) products according to instructions for the MiSeq system (Illumina), followed by quantification using QIAxpert (QIAGEN). Each amplicon was then quantified, set to an equimolar ratio, and sequenced after pooling on a MiSeq platform (Illumina) according to the manufacturer's recommendations. The gut microbiome was profiled using operational taxonomy unit (OTU) clustering and taxonomy profiling with 16S rRNA, 16S rRNA sequence clustering using internationally widely used tools such as UCLUST and QIIME, and 16S rRNA sequence databases such as GreenGenes, RDP, and Silva.

### 1-3. Results of microbiome analysis

Alpha diversity, which represents the species richness of a sample, and beta diversity, which represents the similarity between samples, were analyzed based on results from the microbiome of the normal control group (NL), non-alcoholic fatty liver disease (NAFLD) group, and obese group (OB). Among the alpha diversity indices, ACE is estimation for the species richness based on information on discovered species, taking into account the possibility that species not found or seen in the sample may remain, and Shannon represents a value that estimates the diversity of species present in the sample, meaning that a higher value indicates greater diversity. Comparison was conducted on the microbiome species diversity index in the normal control group (NL), non-alcoholic fatty liver disease (NAFLD) group, and obese group (OB), and based on the comparative results, Linear Discriminant Analysis Effect Size (LEfSe) analysis was followed to identify statistically significant characteristics between specific groups. The Linear Discriminant Analysis Effect Size (LEfSe) analysis was performed using the EZbioCloud program, significant taxa was searched with the <Kruskal-Wallis H test>, and the LDA score was calculated using a difference in the actual relative taxonomic composition values between groups.

As shown in FIG. 1, as a result of comparing the microbiomes of the normal control group (NL) and the non-alcoholic fatty liver disease (NAFLD) group, it was found that the species diversity index was lower in the non-alcoholic fatty liver disease (NAFLD) group compared to the normal control group (NL). Based on the results of the LEfSe analysis, as a result of selecting the taxa for 'species' for animal model evaluation by setting the taxa with a p-value < 0.01 and an LDA effect size >=3 as significant for the non-alcoholic fatty liver disease (NAFLD) group, the microbial species were selected as shown in Table 1 below.

**TABLE 1**

| **Taxon name** | **Taxon rank** | **LDA effect size** | **p-value** | **NL** | **NAFLD** |
|---|---|---|---|---|---|
| *Bacteroides uniformis* | Species | 4.08898 | 0.00888 | 4.534 | 2.3 |
| *Blautia wexlerae* | Species | 4.04632 | 0.00231 | 2.104 | 4.45946 |
| *Alistipes putredinis* | Species | 3.85955 | 0.00137 | 3.102 | 1.43649 |
| PAC001129_s | Species | 3.69169 | 0.02265 | 1.086 | 0.28919 |
| *Ruminococcus gnavus* | Species | 3.5962 | 0.00087 | 0.23 | 1.03311 |
| PAC001654_s | Species | 3.4562 | 0.00005 | 0.59 | 0.01216 |
| *Bacteroides xylanisolvens* group | Species | 3.43864 | 0.00163 | 0.894 | 0.38176 |
| *Blautia hansenii* group | Species | 3.3955 | 0.02881 | 0.186 | 0.64932 |
| *Lactobacillus rogosae* group | Species | 3.38509 | 0.00444 | 0.624 | 0.17432 |
| *Bacteroides intestinalis* | Species | 3.35915 | 0.00062 | 0.48 | 0.0223 |
| *Bacteroides eggerthii* | Species | 3.35614 | 0.0095 | 0.444 | 0.04257 |
| *Bacteroides stercoris* | Species | 3.27378 | 0.04774 | 1.26 | 1.29932 |
| *Coprococcus eutactus* group | Species | 3.27317 | 0.0014 | 0.672 | 0.20338 |
| PAC001040_s | Species | 3.24038 | 0.02321 | 0.586 | 0.26351 |
| PAC001157_s | Species | 3.20479 | 1.213E-07 | 0.38 | 0.06486 |
| *Faecalimonas umbilicata* | Species | 3.20321 | 0.00677 | 0.048 | 0.34054 |
| *Alistipes shahii* | Species | 3.19931 | 0.00825 | 0.572 | 0.23851 |
| JPJG_s | Species | 3.16901 | 0.00869 | 0.524 | 0.23243 |
| PAC001242_s | Species | 3.08047 | 0.00018 | 0.374 | 0.04324 |
| KQ235806_s group | Species | 3.07966 | 0.00709 | 0.376 | 0.16892 |
| PAC000195_s | Species | 3.06671 | 0.00004 | 0.422 | 0.19122 |
| *Ruminococcus bromii* | Species | 3.00945 | 0.028 | 0.9 | 0.85541 |
| *Roseburia intestinalis* group | Species | 3.00488 | 0.01495 | 0.726 | 0.54662 |

As shown in FIG. 2, as a result of comparing the microbiomes of the normal control group (NL) and the obese group (OB), it was found that the species diversity index was lower in the obese group (OB) compared to the normal control group (NL). Based on the results of the LEfSe analysis, as a result of selecting the taxa for 'species' for animal model evaluation by setting the taxa with a p-value < 0.01 and an LDA effect size >=3 as significant for the obese group (OB), the microbial species were selected as shown in Table 2 below.

**TABLE 2**

| **Taxon name** | **Taxon rank** | **LDA effect size** | **p-value** | **NL** | **Obese** |
|---|---|---|---|---|---|
| *Blautia wexlerae* | Species | 4.15736 | 0.00071 | 2.104 | 4.95273 |
| *Bacteroides uniformis* | Species | 3.99853 | 0.01297 | 4.534 | 2.23818 |
| *Alistipes putredinis* | Species | 3.93676 | 0.00172 | 3.102 | 1.43182 |
| *Ruminococcus gnavus* | Species | 3.6756 | 0.00053 | 0.23 | 1.24455 |
| *Faecalibacterium prausnitzii* group | Species | 3.65647 | 0.03407 | 5.478 | 4.62455 |
| PAC001129_s | Species | 3.57996 | 0.01207 | 1.086 | 0.24364 |
| *Bacteroides xylanisolvens* group | Species | 3.51111 | 0.00551 | 0.894 | 0.31636 |
| PAC001654_s | Species | 3.45494 | 0.00004 | 0.59 | 0.01 |
| *Coprococcus eutactus* group | Species | 3.37223 | 0.00526 | 0.672 | 0.23 |
| *Bacteroides intestinalis* | Species | 3.35707 | 0.00083 | 0.48 | 0.01364 |
| *Bacteroides eggerthii* | Species | 3.33873 | 0.02061 | 0.444 | 0.05273 |
| *Lactobacillus rogosae* group | Species | 3.3176 | 0.00432 | 0.624 | 0.18818 |
| *Faecalimonas umbilicata* | Species | 3.26926 | 0.00115 | 0.048 | 0.45 |
| *Blautia hansenii* group | Species | 3.26818 | 0.03174 | 0.186 | 0.53818 |
| HF545616_s | Species | 3.26289 | 0.00409 | 0.39 | 0.10727 |
| PAC001242_s | Species | 3.26115 | 0.0002 | 0.374 | 0.03455 |
| JPJG_s | Species | 3.25067 | 0.00599 | 0.524 | 0.22364 |
| PAC001157_s | Species | 3.24058 | 4.793E-07 | 0.38 | 0.06727 |
| *Alistipes shahii* | Species | 3.21718 | 0.01055 | 0.572 | 0.25455 |
| PAC000195_s | Species | 3.13247 | 1.458E-06 | 0.422 | 0.14909 |
| PAC001040_s | Species | 3.1282 | 0.02416 | 0.586 | 0.3 |
| *Ruminococcus bromii* | Species | 3.11797 | 0.01163 | 0.9 | 0.87636 |
| *Bacteroides cellulosilyticus* | Species | 3.10014 | 0.0006 | 0.322 | 0.09455 |
| PAC001187_s | Species | 3.09594 | 0.00653 | 0.288 | 0.04364 |
| *Gemmiger formicilis* group | Species | 3.08454 | 0.02189 | 0.666 | 0.42182 |
| KQ235806_s group | Species | 3.07906 | 0.02057 | 0.376 | 0.21545 |
| *Eubacterium eligens* group | Species | 3.05039 | 2.059E-07 | 0.346 | 0.13909 |
| PAC001034_s | Species | 3.01735 | 0.04526 | 0.258 | 0.09909 |

From the above results, the strains commonly selected as significant strains in the non-alcoholic fatty liver disease (NAFLD) and obesity (OB) groups compared to the normal control group (NL) were found to be *Bacteroides uniformis, Blautia wexlerae, Alistipes putredinis,* PAC001129_s, *Ruminococcus gnavus,* PAC001654_s, *Bacteroides xylanisolvens* group, *Blautia hansenii* group, *Lactobacillus rogosae* group, *Bacteroides intestinalis, Bacteroides eggerthii , Coprococcus eutactus* group, PAC001040_s, PAC001157_s, *Faecalimonas umbilicata, Alistipes shahii,* JPJG_s, PAC001242_s, KQ235806_s group, PAC000195_s, and *Ruminococcus bromii.* Among these, *Bacteroides eggerthii* was selected as the final microbiome marker.

### Example 2. Evaluation of efficacy of Bacteroides eggerthii KGMB05336

### 2-1. Culture of Bacteroides eggerthii

Based on the results of Example 1 above, in order to determine whether *Bacteroides eggerthii* is a strain effective for obesity or non-alcoholic fatty liver disease, a commercially available *Bacteroides eggerthii* strain, *Bacteroides eggerthii* KGMB05336, was purchased from the Korean Gut Microbiome Bank (KGMB). The *Bacteroides eggerthii* KGMB05336 strain obtained was inoculated into a medium supplemented with tryptic soy agar (TSA, MB-T0689) and 5% horse blood defibrinated (HBD, MB-H1883), and anaerobically cultured at 37°C in an anaerobic jar having an MGC anaerobic pack. The number of viable cells was measured based on the OD₆₀₀ value to determine CFU/mL. *Bacteroides eggerthii* KGMB05336 was deposited at the Korea Research Institute of Bioscience and Biotechnology (Korean Collection for Type Cultures) on August 29, 2023, and the Accession No. KCTC15573BP was issued.

### 2-2. Animal model with a non-alcoholic fatty liver disease (NAFLD)

To determine whether the *Bacteroides eggerthii* KGMB05336 strain exhibits an improving effect on obesity and non-alcoholic fatty liver disease caused by obesity, an animal model with an onset of the non-alcoholic fatty liver disease (NAFLD) caused by obesity was established (FIG. 3). Five-week-old C57BL/6N male mice were adopted and fed a normal diet for one week to allow them to adapt to the breeding space. After dividing the mice into three groups, the normal diet group (ND) was fed a normal diet, and the negative control group and experimental group were fed a Western diet (TD88137, Dooyeol biotech, Seoul, Korea) to induce obesity. The Western diet, which is a diet with a high content of fat, sugar, and cholesterol, is used to induce diseases such as obesity, non-alcoholic steatohepatitis, and diabetes in mouse animal models. The Western diet had a composition consisting of 42% fat, 42.7% carbohydrate, and 15% protein, and all mice were allowed to drink water freely. The normal diet group (ND) was fed only a normal diet, the negative control group (WD+PBS) was fed a Western diet and orally administered 200 µL of PBS 5 times/week for 12 weeks, and the experimental group (WD+B. eggerttii) was fed a Western diet and orally administered 200 µL of the *Bacteroides eggerthii* KGMB05336 strain diluted in PBS at a concentration of 10⁸ CFU/mouse 5 times/week for 12 weeks. The body weights of mice were measured once a week after the start of oral administration.

After the experiment was completed and before necropsy of the mice, blood from each mouse was collected by orbital blood collection using a heparinized capillary tube, the collected blood was centrifuged at 3,000 rpm for 10 minutes to collect serum, and the collected serum was then stored at -80°C. On the day of autopsy, the body weight was measured, laparotomy images were taken, liver tissue was removed, photographed, and then preserved in 10% neutral buffered formalin, followed by histopathological examination (H&E, Masson's trichrome staining).

### 2-3. Identification of an efficacy of Bacteroides eggerthii in an animal model with a fatty liver disease

As a result of observing the weight changes up to week 13 as shown in FIG. 4, the negative control group (WD+PBS) and experimental group (WD+B.eggerttii) fed a Western diet showed statistically significant weight gain compared to the normal diet group (ND) fed a normal diet. However, the experimental group (WD+B.eggerttii) that was fed a Western diet and administered *Bacteroides eggerthii* showed a significant decrease in body weight compared to the negative control group (WD+PBS) that was fed only a Western diet. The results demonstrate that diet-induced obesity is treated and ameliorated by administration of *Bacteroides eggerthii .*

As a result of measuring the liver weight as shown in FIG. 5, the negative control group (WD+PBS) and the experimental group (WD+B.eggerttii) fed a Western diet showed a statistically significant increase in the liver weight compared to the normal diet group (ND) fed a normal diet. However, in the experimental group (WD+B.eggerttii) that was fed a Western diet and administered *Bacteroides eggerthii* , the liver weight was significantly reduced compared to the negative control group (WD+PBS) that was fed only a Western diet. The results demonstrate that diet-induced obesity and obesity-induced hepatomegaly are treated and ameliorated by administration of *Bacteroides eggerthii .*

As a result of opening the abdomen of mice with the experiment completed to observe the fat level, as shown in FIG. 6, the fat level was found to have increased significantly in the negative control group (WD+PBS) and experimental group (WD+B.eggerttii) fed a Western diet compared to the normal diet group (ND) fed a normal diet. However, in the experimental group (WD+B.eggerttii) that was fed a Western diet and administered *Bacteroides eggerthii* , the degree of fat accumulation in the intestines was found to be less increased than in the negative control group (WD+PBS) that was fed only a Western diet. The results demonstrate that diet-induced obesity is treated and ameliorated by administration of *Bacteroides eggerthii .*

In addition, as a result of observing liver color of the mouse, as shown in FIG. 7, when fat accumulates in the liver, the color turns yellow and the liver becomes enlarged; compared to the normal diet group (ND) fed a normal diet, the liver color of the negative control group (WD+PBS) fed a Western diet turned yellow and had a significant increase in size. However, in the experimental group (WD+B.eggerttii) that was fed a Western diet and administered *Bacteroides eggerthii* , the liver color was found to be close to normal and the size was relatively similar to normal compared to the negative control group (WD+PBS) that was fed only a Western diet. The above results demonstrate that diet-induced obesity, obesity-induced fatty liver and hepatomegaly are treated and improved by administration of *Bacteroides eggerthii .*

### 2-4. Evaluation of pathological changes in liver tissues

The effect of the *Bacteroides eggerthii* KGMB05336 strain on liver tissue pathology was evaluated. Liver tissues from mouse models of the control group fed a normal diet (ND), the experimental group fed a Western diet (Western diet + PBS, WD), and the experimental group fed a Western diet plus the *Bacteroides eggerthii* KGMB05336 strain (WD + Strain) were collected, stained with H&E, and analyzed. As shown in FIG. 8, it was confirmed that fat accumulation in hepatocytes was aggravated in the experimental group fed a Western diet, but fat accumulation was found to be improved in the experimental group fed the *Bacteroides eggerthii* KGMB05336 strain. In addition, as a result of evaluating the non-alcoholic fatty liver disease activity score (NAS), NAS increased and worsened in the experimental group which fed a Western diet, but the level of NAS decreased in the experimental group fed the *Bacteroides eggerthii* KGMB05336 strain. In addition, as a result of comparing the degree of fibrosis, no fibrosis was observed at all in the normal diet group (ND), but in the experimental group fed a Western diet (WD), stages 1a, 1b, and 2 were observed in 10 mice, 3 mice, and 1 mouse, respectively, whereas in the experimental group fed with the *Bacteroides eggerthii* KGMB05336 strain (WD+Stain), stages 1a and 1b were observed in 8 mice and 1 mouse, respectively, presenting an improvement in fibrosis.

### 2-5. Results of blood test

To evaluate the effects of the *Bacteroides eggerthii* KGMB05336 strain on plasma factors, blood tests were performed to analyze changes in the levels of aspartate aminotransferase (AST), alanine transaminase (ALT), total bilirubin, glucose, cholesterol, triglyceride, high density lipoprotein (HDL) cholesterol, and low density lipoprotein (LDL) cholesterol that are related to liver function. As shown in FIG. 9, in the experimental group (WD) fed a Western diet, AST, ALT, cholesterol, blood glucose, triglyceride, HDL, and LDL levels were found to increase significantly compared to the control group (ND). On the other hand, in the experimental group (WD+B) fed the *Bacteroides eggerthii* KGMB05336 strain, the levels of AST, ALT, cholesterol, blood glucose, triglyceride, HDL, and LDL, which increased with a Western diet, were significantly reduced.

For the non-alcoholic fatty liver disease, levels of serum AST (U/L) and ALT (U/L) are increased, based on which the risk of the non-alcoholic fatty liver disease is measured by serum AST (U/L) and ALT (U/L) levels. The results showed that the levels of serum AST (U/L) and ALT (U/L), which increased by a Western diet, were reduced by administering *Bacteroides eggerthii* , which demonstrates that *Bacteroides eggerthii* has a preventive, therapeutic, and ameliorating effect on the non-alcoholic fatty liver disease induced by obesity. Additionally, the risk of diabetes is measured by levels of cholesterol (mg/dL) and glucose (mg/dL). The results showed that the levels of cholesterol (mg/dL) and glucose (mg/dL), which increased by a Western diet, were reduced by administering *Bacteroides eggerthii ,* which demonstrates that *Bacteroides eggerthii* has a preventive, therapeutic, and ameliorating effect on diabetes induced by obesity.

### 2-6. Analysis of transcriptome in liver tissues

To evaluate the effect of the *Bacteroides eggerthii* KGMB05336 strain on changes in the liver tissue microbiota, the transcriptome in the liver tissue was analyzed. Microbiome analysis was performed using mouse feces, and transcriptome analysis was carried out using mouse liver tissue, followed by analysis through integration of the two types of omics data.

In the experimental group fed a Western diet (WD), analysis was made on the correlation between candidate genes whose expression increased and then decreased upon feeding with the *Bacteroides eggerthii* KGMB05336 strain and expression of microbiome mRNA. As shown in FIG. 10, among the 117 candidate genes whose expression increased when fed a Western diet but decreased when fed the *Bacteroides eggerthii* KGMB05336 strain, 82 genes were confirmed to have a positive or negative correlation with microbiome mRNA expression.

Additionally, analysis was conducted on the correlation between candidate genes whose expression decreased in the experimental group fed a Western diet (WD) and then increased upon feeding with the *Bacteroides eggerthii* KGMB05336 strain and expression of microbiome mRNA. As shown in FIG. 11, among 105 candidate genes whose expression decreased when fed a Western diet but increased when fed the *Bacteroides eggerthii* KGMB05336 strain, 68 genes were confirmed to have a positive or negative correlation in the microbiome mRNA expression.

### 2-7. Analysis of a network of Microbiome and metabolites

To analyze the effects of the *Bacteroides eggerthii* KGMB05336 strain on changes in the microbiome and metabolite, primary metabolites and bile acids were analyzed using feces, and then the correlation with the fecal microbiome was analyzed.

Analysis was conducted on the network of metabolites related to the microbiome, which decreased in the experimental group fed a Western diet (WD), but increased when fed the *Bacteroides eggerthii* KGMB05336 strain. As shown in FIG. 12, in Bacteroides, it was found that 2-hydroxylsocaproic acid showed a positive correlation, and 1-hexadecanol and 1-pentadecanol showed a negative correlation; and in Lactobacillus, gamma-tocopherol, 1,2-propanediol, delta-tocopherol, linoleic acid, 1-monomyristin, 3-hydroxybutyric acid and phosphoric acid, 5-nonadecylresorcinol, pyrimidine, and xylose showed representative positive correlations; and in the KE159538_g strain, palmitic acid, 5-heptadecylresorcinol, and phloretic acid showed a positive correlation. *Bacteroides eggerthii* appears to be involved in the metabolic pathway of 2-hydroxysocaproic acid or promotes its production. This appears to be consistent with previous research showing that some gut bacteria may produce organic acids or regulate their levels. Additionally, the negative correlations with 1-hexadecanol and 1-pentadecanol suggest that supplying *Bacteroides eggerthii* may utilize these compounds or inhibit the production thereof. Fatty alcohols are involved in lipid metabolism, and changes in their levels may reflect changes in the metabolism of gut microbiota and lipid absorption.

Additionally, analysis was conducted on the network of metabolites related to the microbiome, which increased in the experimental group fed a Western diet (WD) but decreased when fed the *Bacteroides eggerthii* KGMB05336 strain. As shown in FIG. 13, KE159571_g, Mucispirillum, KE159600_g, and Alistipes strains were found to have positive or negative correlations with 20 fatty acids, 2 carbohydrates, and 4 other metabolites. KE159600_g showed a negative correlation with xylose, and KE159600_g or Alistipes showed a positive correlation with ferulic acid and also with most fatty acids. The correlation observed between specific gut microbes and metabolites suggests that their metabolic activity has impact on the production or consumption of metabolites. For example, Alistipes shows positive correlations with various fatty acids, indicating that it may play an important role in fatty acid metabolism.

In conclusion, the above results demonstrate that *Bacteroides eggerthii* has preventive, therapeutic, and ameliorating effects on diet-induced obesity as well as obesity-induced fatty liver, hepatomegaly, non-alcoholic fatty liver disease, and diabetes.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

The numerical range includes the numerical values defined in the above range. All maximum numerical limits given throughout the specification include all lower numerical limits as if the lower numerical limits were expressly written. All minimum numerical limits given throughout this specification include all higher numerical limits as if the higher numerical limits were expressly written. All numerical limits given throughout this specification will include all the better numerical range within the broader numerical range, as if the narrower numerical limits were explicitly written.

### [Microorganism Deposit]

Depositary Institution: Korea Research Institute of Bioscience and Biotechnology (Korean Collection for Type Cultures)
Accession No.: KCTC15573BP
Date of Deposit: August 29, 2023.

## Claims

1. A pharmaceutical composition for preventing or treating obesity or an obesity-induced disease, comprising the *Bacteroides eggerthii* KGMB05336 strain having Accession No. KCTC15573BP, or a culture or dried product thereof.

2. The pharmaceutical composition of claim 1, wherein the obesity-induced disease is one or more selected from the group consisting of fatty liver, hepatomegaly, non-alcoholic fatty liver disease, and diabetes.

3. The pharmaceutical composition of claim 1, wherein the pharmaceutical composition reduces body weight upon oral administration.

4. The pharmaceutical composition of claim 1, wherein the pharmaceutical composition ameliorates fatty liver upon oral administration.

5. The pharmaceutical composition of claim 1, wherein the pharmaceutical composition improves serum levels of AST (U/L), ALT (U/L), cholesterol (mg/dL), and glucose (mg/dL) upon oral administration.

6. A probiotic composition for preventing or ameliorating obesity or an obesity-induced disease, comprising the *Bacteroides eggerthii* KGMB05336 strain having Accession No. KCTC15573BP, or a culture or dried product thereof.

7. The probiotic composition of claim 6, wherein the obesity-induced disease is one or more selected from the group consisting of fatty liver, hepatomegaly, non-alcoholic fatty liver disease, and diabetes.

8. A health functional food composition for preventing or ameliorating obesity or an obesity-induced disease, comprising the *Bacteroides eggerthii* KGMB05336 strain having Accession No. KCTC15573BP, or a culture or dried product thereof.

9. The health functional food composition of claim 8, wherein the obesity-induced disease is one or more selected from the group consisting of fatty liver, hepatomegaly, non-alcoholic fatty liver disease, and diabetes.
